# EUROPEAN PATENT SPECIFICATION

(11) **EP 1 727 421 B1**
(45) Date of publication and mention of the grant of the patent: **31.03.2010**
(21) Application number: 04789066.0
(22) Date of filing: 27.09.2004
(51) Int. Cl.: A01K 13/00

(54) **ANIMAL COVER HAVING A TEMPERATURE ALTERING DEVICE**
TIERABDECKUNG MIT TEMPERATURÄNDERUNGSVORRICHTUNG
COUVERTURE POUR ANIMAUX PRESENTANT UN DISPOSITIF DE MODIFICATION DE LA TEMPERATURE

(30) Priority: 24.03.2004 US 807695
(43) Date of publication of application: 06.12.2006
(73) Proprietor: Recover Blankets, LLC, Cardiff-by-the-Sea, CA 92007 (US)
(72) Inventor: NILFORUSHAN, Ali, Cardiff-by-the-Sea, CA 92007 (US)
(74) Representative: Dr. Graf & Partner
(86) International application number: PCT/US2004/031540
(87) International publication number: WO 2005/102341

(56) References cited:
- DE-U1- 20 021 260
- GB-A- 2 135 572
- GB-A- 2 374 535
- JP-A- 10 113 088
- US-A- 4 592 358
- US-A- 5 398 667
- US-A- 5 537 954
- US-A- 6 128 891

## Description

### FIELD OF THE INVENTION

This invention relates generally to the field of animal care, and particularly to delivering a temperature altering regimen to a specific and defined location on an animal's body. A temperature altering system is known from DE - 200 21 260 U.

### BACKGROUND

Covers, blankets, clothing and other articles adapted for wearing by an animal have long been used. Common examples include, clothing for dogs, fly protectors (used to prevent or lessen damage to cattle and other farm animals caused by ectoparasites, such as flies and mosquitoes) and horse blankets. Conventional horse blankets are provided to protect a horse from mud, dirt, and moisture, and to provide a degree of thermal insulation. These conventional horse blankets typically conform to the shape of a horse's upper body to provide adequate protection from the elements.

The horse blankets of the prior art provides a general protection from the elements; however, the prior art is deficient in delivering a targeted extreme temperature to a specifically focused area of the horse's body. Similarly, other animal covers and clothing merely provide a general barrier between the animal and the elements. Furthermore, the animal covers of the prior art are not useful for delivering an extreme temperature regimen to the animal's body, or to a defined area thereof Thus, there is a need in the art for an animal cover that delivers targeted and extreme temperature to a specifically selected area of an animal's body, or to the entire body.

### SUMMARY OF THE INVENTION

In the present invention as set out in claim 1, a horse blanket is provided and is used to deliver a temperature altering regimen to a competition horse. The invention comprises strategically located cavities further comprising a temperature altering device. The horse blanket, therefore, places the temperature altering device in a specific and defined location on the horse's body wherein a temperature altering regimen is delivered.

In the present invention, the horse blanket is having at least one cavity releasably attached to said horse blanket.

The blanket is placed on the horse and then a cavity containing a temperature altering device is attached to the blanket such that the cavity can deliver a temperature altering regimen to a desired location of the horse's body, including, but not limited to muscle groups, joints, ligaments and tendons. The cavity is later removed from the blanket and can be reattached in the same or in another location on the horse blanket.

### BRIEF DESCRIPTION OF THE DRAWINGS

Figure 1A shows the horse blanket embodiment of the current invention; wherein the horse blanket is laid open.
Figures 1B-D illustrate alternative configurations not forming part of the present invention for placing the cavity on a horse blanket. The cavity further comprises a temperature altering device, thus their placement will define the region of the blanket that will deliver a temperature altering regimen.
Figure 1E shows the horse blanket embodiment of the current invention, with cavities placed in specifically selected spots on the horse blanket and with an optional neck piece connected to the said blanket, wherein the horse blanket is shown covering the body of a horse.
Figure 2A is a cross sectional view of a cavity embedded into a horse blanket.
Figure 2B is a view of one embodiment for placement of an adjustable cavity in a horse blanket, specifically in this illustration on the flap of the horse blanket body.
Figure 2C is a view of one alternative embodiment for placement of an adjustable cavity in a horse blanket, specifically in this illustration on the flap of the horse blanket body.
Figure 3 is a cross sectional view of a cavity having a sealable pocket mouth for removably placing a temperature altering device.
Figure 4a illustrates an alternative embodiment of the invention wherein the interior side of the animal cover comprises a material for the releasable attachment of cavities to the animal cover. Also seen in figure 4a is an alternative embodiment of flap 12 wherein the flap is releasably connected near the buttock and near the hip areas of the animal.
Figure 4b illustrates an embodiment wherein the exterior side of the animal cover comprises a material for the releasable attachment of cavities to the animal cover.
Figure 5a illustrates a releasable cavity of the current invention wherein the cavity is a pocket comprising releasable material, temperature reflective material and temperature permeable material, and further comprising a pocket for holding a temperature altering device.
Figure 5b illustrates a releasable cavity of the current invention wherein the cavity is a patch comprising releasable material and comprising either a temperature reflective material or temperature permeable material, wherein the patch can envelop a temperature altering device when used in conjunction with an animal cover having the complementary releasable material.
Figure 6 is an illustration of flap 12 in an alternative embodiment wherein the entire flap is separable from the remainder of the animal cover body.

### DETAILED DESCRIPTION OF THE INVENTION

The current invention is a cover used to alter the temperature of a user. Preferably, the user referred to herein is an animal, more preferably, the user is a competition animal and most preferably, the user is a competition horse. For the remainder of this description of the current invention, a horse blanket will be described; however, those of ordinary skill in the art will readily adapt the current invention to a variety of covers for a variety of animals. These additional uses of the current invention, including alternative embodiments involving different types of covers, different temperature altering devices or different animals, are well within the scope of the current invention.

It is desirable to alter the temperature of an animal for a variety of reasons. Veterinarians typically use ice baths to lower the temperature of an animal suffering from a fever and will use a hot or cold press to deliver a temperature to a specific location on an animal's body. Similarly, farmers and other animal owners, particularly those in more extreme climates, desire to alter the body temperature of their animals to prevent or ameliorate hypothermia, hyperthermia, and other adverse conditions associated with exposure to extreme climates. Thus, animal blankets are frequently used on animals in extremely cold climates to keep these animals warm.

Competition animals exert maximum effort during a performance or practice, and as a result, will often develop sore muscles, inflamed joints, strained ligaments and tendons, increased body temperature and a variety of other undesirable effects. Such competition animals include, but are not limited to greyhound race dogs, thoroughbred race horses, sport horses (e.g., jumping, driving, dressage, vaulting and endurance) and agility dogs. It is desirable to deliver a specific and targeted temperature altering treatment to one or more of the sore muscles, inflamed joints, strained ligaments and tendons, increased body temperature and variety of other undesirable effects. In addition to targeting a specific area with a temperature altering regimen, it may also be desirable to deliver a first specific temperature to a first specific area and a second specific temperature to a second specific area. These and other objectives are achieved by the current invention.

The current invention is directed towards a new device and method for delivering a temperature altering regimen to an animal. In the preferred embodiment, the animal is a competitions horse. The device in this embodiment is a cover, similar to a horse blanket that covers the body of the horse and delivers a temperature altering regimen directly to a specific location on the horse's body. The device of the current invention is adjustable, allowing the temperature altering regions to precisely fit a variety of body areas of various differently shaped and sized horses. The device and method is further described below with reference to the attached drawings.

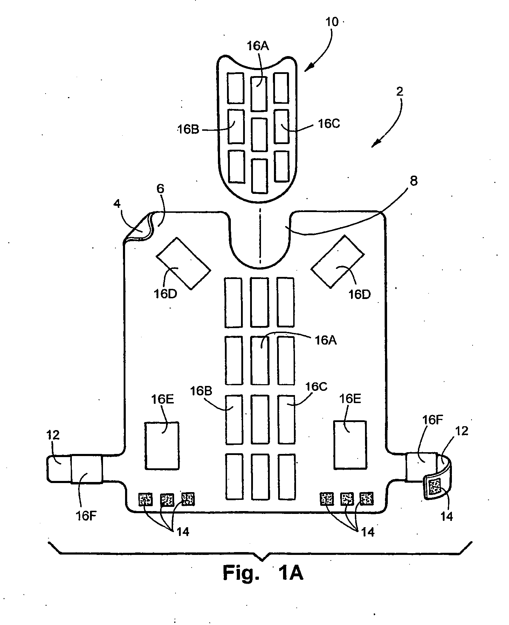

Figure 1A is a horse blanket 2, having an interior side 4 and an exterior side 6. The interior side 4 refers to the side of the blanket that is closest to the horse's body. In the preferred embodiment, the material of interior side 4 of horse blanket 2 is constructed from a material that will wick moisture away from the body of the horse, thereby preventing rash and other problems associated with having moisture trapped against the animal's body. In the preferred embodiment, the material of exterior side 6 of horse blanket 2 is constructed from a material that will reflect temperature, thereby directing a temperature altering regimen towards the body of the animal for a maximally efficient delivery of said temperature altering regimen.

In an alternative embodiment, the material of horse blanket 2 is cotton or a cotton-polyester blend on one or both of the interior side 4 and the exterior side 6. At times it may be desirable for the horse blanket 2 to possess certain specific qualities or a combination thereof, including but not limited to a variable degree of flexibility, water impermeability, tensile strength, optical reflectivity, conductivity and elasticity. In a further alternative embodiment, the material comprising one or both of interior side 4 and exterior side 6 is a specialty material selected from the group consisting of polychloroprene, polyethylene, polyvinylidene chloride, polyacrylonitrile, polystyrene and polyamide.

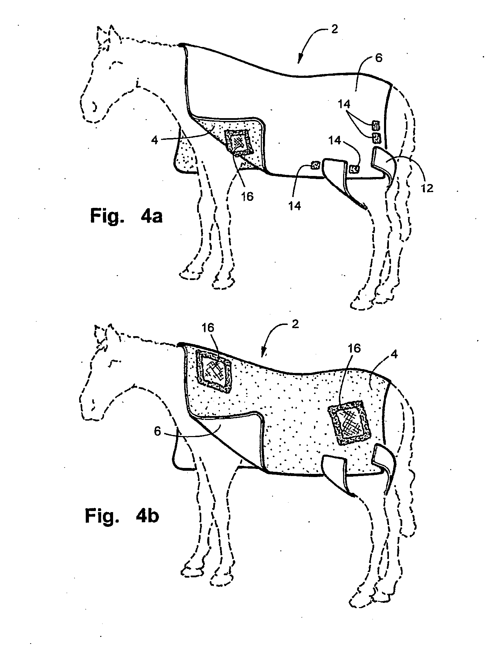

In another embodiment, the material of horse blanket 2 is comprises a loop material, such that various objects comprising a hook component are releasably attachable to various locations on the horse blanket 2. In one example configuration of this alternative embodiment, the material of interior side 4 of blanket 2 is made of loop material and the exterior side 6 of blanket 2 is made of a temperature reflective material. (Figure 4a.) It is well known that the loop material of a hook and loop is generally the less abrasive of the materials and therefore will not irritate the horse's skin. As will be described below in further detail, the temperature altering devices of the current invention are enclosed in a cavity comprising a hook material and, therefore, are releasably attached to a variety of locations on interior side 4 of blanket 2. In figure 4a, the releasably attached cavity comprising a temperature altering device is generally labeled 16.

In another example configuration of this alternative embodiment, the material of exterior side 6 of blanket 2 is made of loop material. (Figure 4b.) As will be described below in further detail, the temperature altering devices of the current invention are enclosed in a cavity comprising a hook material and, therefore, are releasably attached to a variety of locations on exterior side 6 of blanket 2. Again, the releasably attached cavity comprising a temperature altering device is generally labeled 16. In this example configuration, blanket 2 is made of a material that is permeable to a temperature altering regimen, and that can, preferably, wick moisture away form the horse's body. In a further aspect of this example configuration, the cavity comprising the temperature altering device comprises a temperature reflective cover on the outermost surface, thereby directing the temperature altering regimen through the blanket 2 and towards the horse's body.

The animal cover, which is a horse blanket 2 in the current description, can thusly, comprise a variety of materials, including, but not limited to, hook material, loop material, temperature permeable material, temperature reflective material, moisture wicking material and combinations thereof.

The front end of the horse blanket comprises a cut away portion 8 designed to fit around the horse's neck, resting just above the withers. Alternatively seen as a detached section, the horse blanket 2 comprises a protrusion 10 that covers a majority of the horse's neck, stopping just short of the horse's head. The rear sides of the horse blanket comprises flaps 12, which are fitted around the hips of the horse and releasably attached to connectors 14 at the rear of the horse blanket. Connectors 14 can be a variety of well known connectors, including, but not limited to, hook and loop, snaps, ring and clip, and buckle. Furthermore, connectors 14 can be a plurality of connectors 14 thereby allowing for an adjustable attachment of the horse blanket 2 to a variety of sized horses. The horse blanket is attached to the horse's body using fastening systems well known in the art.

Also shown in figure 1A horse blanket 2 comprises a plurality of cavities 16. The cavities 16 enclose the temperature altering device (not shown) and are placed at a determined position within the horse blanket 2. The cavities 16 are releasably attached to horse blanket 2.

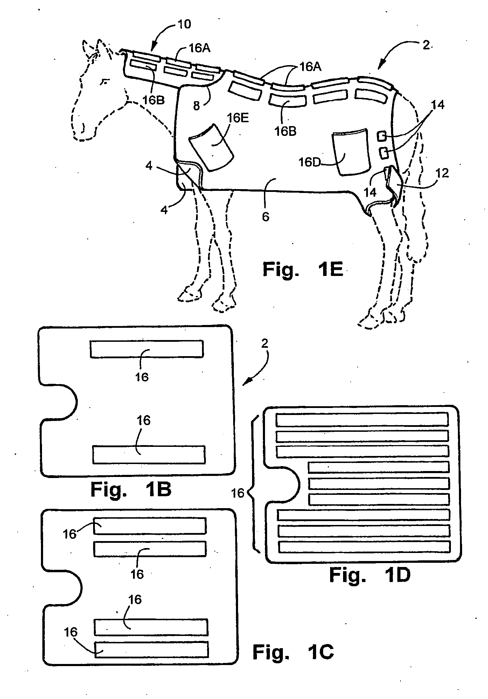

In Figure 1a cavities 16a-c are strategically located within blanket 2 to contact the horse's spine 16a and spinal muscles 16b-c. Cavities 16d are strategically located within blanket 2 to contact the horse's shoulder muscles. Cavities 16e are strategically located within blanket 2 to contact the horse's hip muscles. Cavities 16f are strategically located within blanket 2 to contact the horse's stifle joint. In an alternative embodiment wherein the horse blanket further comprises a protrusion 10 covering the horse's neck, cavities 16a-c may also extend through the protrusion 10 making contact with the horse's c-spine and associated muscles. Figure 1E illustrates the invention horse blanket 2 on a horse and having cavities 16 strategically located along the spine, spine muscles, shoulder and hips. Figure 1E is an example only, and does not limit the current invention to a horse blanket or to having cavities in the illustrated locations.

Cavities 16 can be located in any portions of horse blanket 2 that is desired. For example, in figure 1B, the cavities are shown strategically located within the horse blanket 2 to contact the horse's thoracic cavity. If the horse blanket 2 is designed such that it is capable of attaching around the underside of a horse, then the cavities 16 can be strategically located to contact the horse's girth and abdomen, as is shown in figure 1C. In a further embodiment of the current invention, the cavities 16 are located throughout the entire horse blanket (figure 1D).

These and other embodiments for locating these cavities in an animal cover are well within the scope of this current invention. A hook and loop material is used as the releasably attachment mechanism.

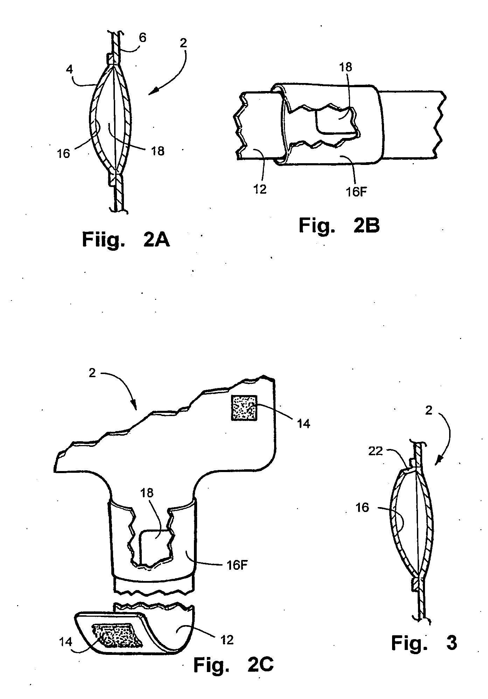

Cavities 16 are designed to hold the temperature altering device of the current invention via enclosing said temperature altering device within said cavities 16. In figure 2A, a cavity of the current invention is illustrated in cross sectional view. In a preferred embodiment, horse blanket 2, comprises interior side 4; exterior side 6 and cavity 16. Preferably, interior side 4 of horse blanket 2 is made of a material that wicks moisture away from the horse's body. The material of the horse blanket 2 forming cavity 16, however, preferably reflects the temperature from the temperature altering device 18 towards the body of the horse, thereby delivering an efficient temperature altering regimen to that location of the body. In this aspect, cavity 16 is surrounded by a temperature reflective material on the exterior 6 side of the horse blanket 2, and a temperature permeable material on the interior 4 side of horse blanket 2.

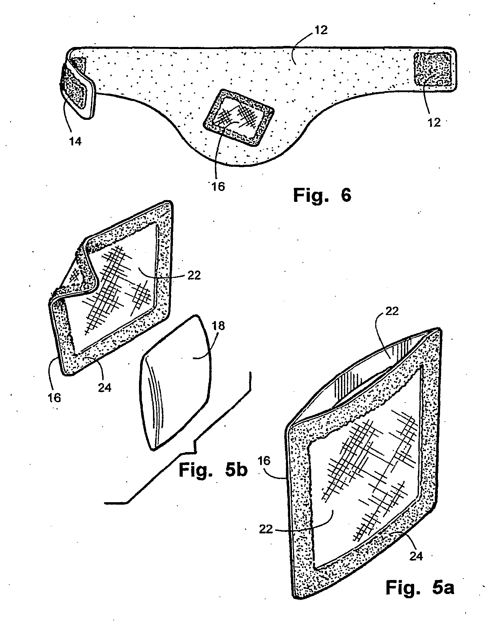

The horse blanket 2 comprises a loop material forming the interior side 4 or the exterior side 6, the cavities 16 are constructed separately form the horse blanket 2 and made to releasably attach with horse blanket 2. For example, wherein the interior side 4 of horse blanket 2 is a loop material, a cavity 16 of this embodiment is illustrated in figure 5a and comprises a first surface 22 surrounded by a hook material boarder 24. In this embodiment, the first surface is preferably a temperature reflective material and the second surface 26 (not shown), which is opposite said first surface 22 and hook material boarder 24, is preferably a temperature permeable material. Conversely, wherein the exterior side 6 of horse blanket 2 is a loop material, the first surface 22 is temperature permeable and the second surface 26 is temperature reflective. The temperature reflective surface can be slightly larger in area that the temperature permeable area, thereby preventing temperature escape from the edges of temperature permeable area.

Alternatively, and as shown in figure 5b, cavities 16 are designed as a patch that forms a cavity with either interior side 4 or exterior side 6 of horse blanket 2, depending on which side comprises the complementary hook or loop material. For example, when the interior side 4 of horse blanket 2 comprises a loop material, then a cavity 16 comprises a first surface 22 that is temperature permeable and comprises a hook material boarder 24. The total area of first surface 22 and hook material 24 must be sufficient to envelop a temperature altering device 18 (described in detail below) when connected to the loop material of interior side 4. In this example, exterior side 6 will preferably comprise a temperature reflective material.

In an example wherein the exterior side 6 of blanket 2 comprises a loop material, then cavity 16 is formed as in the example directly above; however, in this example, the first surface 22 is temperature reflective and the material comprising horse blanket 2 is temperature permeable.

As is illustrated in figure 1a, horse blanket 2 can comprise flap 12 which is fitted around the hips of the horse and connect to the rear of horse blanket 2 using connectors 14. (See also figure 1e showing the flap 12 in use.) Furthermore, cavity 16f can be permanently or releasably located on the flap 12 of horse blanket 2 and deliver a temperature altering regimen to the stifle joint, inner thigh, groin and/or other anatomical parts of the horse that are positioned in that region.

For example, flap 12 will generally extend from horse blanket 2 near the front of the horse's thigh/point of hip region. Flap 12 can be placed behind the horse's thigh and releasably connect to the horse blanket near the rear of thigh/buttock region of the horse. Preferably, the releasable connection 14 is a spring clamp and loop assembly and more preferably a hook and loop connection; however, a variety of other connector mechanisms are well known in the art and can be used in this embodiment. The connector 14 is also adjustable, allowing for a conforming proper fit for flap 12 with variously sized and shaped horses. As used with the current invention, flap 12 comprises a cavity 16f for delivering a temperature altering regimen to a horse. (See figures 1a and 1e.)

In one preferred embodiment, cavity 16f is adjustably attached along flap 12 for proper positioning of the temperature altering device 18. For example, as is shown in figure 2B, the cavity 16f is formed as a separate, unit that is slidable along the longitude of flap 12. In this example, the flap 12 is positioned as described above. Cavity 16f is circular forming a lumen through which flap 12 traverses. The lumen formed by cavity 16f is just slightly larger than the cross area of flap 12, which is encircles. Cavity 16f further comprises a temperature altering device that, when flap 12 is positioned behind the horse's thigh, is positioned to deliver a temperature altering regimen to a specific area of the thigh. That specific area can be very precisely targeted by traversing cavity 16f longitudinally along flap 12 and/or by rotating cavity 16f around flap 12. Once the desired area of the horse is in contact with the temperature altering device 18 within cavity 16f, flap 12 is securely fastened using connectors 14. It is preferred that the material forming cavity 16f is temperature permeable.

In an alternative embodiment, the cavity 16f is formed within flap 12 as described above for cavities 16a-e; however, in this example cavity 16f is much larger than the temperature altering device 18 (Figure 2C) and thereby allows for the temperature altering device to move within said cavity 16f. (Temperature altering device 18 is detailed below.) In this alternative embodiment, the temperature altering device is moveable within flap 12, and is secured at a desired position using a clamping mechanism, or by tightly connecting flap 12 to the remainder of the horse blanket 2 using connectors 14.

In a still further alternative embodiment, flap 12 comprises a loop material and cavity 16f comprises a hook material. As is detailed above, the cavity 16f is then releasably attached to flap 12 using the hook and loop attachment material. Cavity 16f is then brought into position and delivers a temperature altering regimen as described above.

In a further embodiment illustrated in figure 6, flap 12 is a member separate from the remaining body of the horse blanket 2. In this embodiment, flap 12 comprises connectors 14 on both ends and the connectors 14 attach to their complementary connector 14 at the buttock end of the horse blanket 2 and at the thigh/point of hip region on the other end. (See figure 4a for attachment illustration.). In this example, the detached flap member further comprises a cavity 16 having a temperature altering device 18, as described herein, and the cavity 16 is placed in a specific location on the horse and then the connectors 14 are secured. This will place the cavity 16, and in turn the temperature altering device 18, in contact with a specific location in the horse body.

Other embodiments will be apparent to those of ordinary skill in the art and do not exceed the current invention. In addition, the positional cavity and/or temperature altering device is not limited to that on flap 12, as is apparent to those of ordinary skill in the art.

Temperature altering device 18 delivers a desired temperature to a specific area of a horse's body. The temperature altering device 18 of the current invention is strategically located to deliver a temperature altering regimen to an optimal location of an animal's body. The temperature altering device 18 is typically located in a cavity 16 of the current invention.

The animal cover has releasably attached cavities that can be strategically placed on said animal cover, and thereby optimally located to deliver a temperature altering regimen to an animal's body. Releasable attachment means are discussed above, are well known in the art, and are within the spirit of the current invention. Animal covers of this embodiment can come in a variety of sizes to fit a variety of animal types and sizes; however, optimal placement of said cavities is achieved by the releasable attachment of said cavities.

As stated above, the temperature altering device 18 delivers a desired temperature to a specific area of a horse's body. The delivered temperature can be either greater than or lesser than the horse's current basal temperature. Following a strenuous workout the horse's body will increase its temperature relative to basal temperature. In this example, the current invention comprises a cooling temperature altering device 18, which can be applied to the horse's body to help the horse cool down. Horses' muscles and joints will often require heat or cold, and this is particularly true following a strenuous workout or an injury. In these instances, the current invention comprises either a heating or a cooling temperature altering device 18.

A temperature altering device 18 is located within a cavity 16. The temperature altering device 18 can either be permanently located within cavity 16 or removably located within cavity 16. If the temperature altering device 18 is permanently located within cavity 16, then it can be sown within the fabric, or otherwise included within. If; however, the temperature altering device 18 is removably located within cavity 16, then cavity 16 can be in the form of a pocket, having a securable pocket mouth 22 (figure 3 and figure 5a).

Securable pocket mouth 22 can be closed using a variety of means well known in the art. It is preferable that the securing means for securable pocket mouth 22 are such that the entire length of securable pocket mouth 22 is sealed, thereby preventing a loss of temperature from temperature altering device 18. Securable pocket mouth 22 can be closed with a variety of securing means, including but not limited to: hook and loop, snap, zipper, button, tuck, or a variety of other means well know in the art.

Horse blanket 2 can comprise temperature altering device 18 removably located in cavity 16. In this embodiment, the temperature altering device 18 can be a heating or cooling element, a gel pack similar to that used in heat and cool packs, a gel, silica, water, bean bags, rice bags and other compositions for altering temperature. These temperature altering devices can be electrically connected to a power source (for the heating or cooling element) or can be placed in a temperature altering environment, brought to a desired temperature through physical or chemical means and then returned to horse blanket 2 to deliver a temperature altering regimen. If a heat pack or cool pack is used, then submersion in hot or cold water is one means for adjusting the temperature of said temperature altering device 18, because only the packs and not the whole blanket will be submerged in water. Temperature altering device 18 can be a single use device or material. For example, cavity 16 can be filled with ice, or with an activated disposable heat or cool pack (e.g., those containing sodium chloride and water for heat, or those containing ammonium nitrate and water for cold). If a heat pack or cool pack is used, another means for adjusting the temperature of said temperature altering device 18 is filling the heat pack or cool pack with gel or other medium, wherein such gel or medium can be activated to undergo an exothermic or endothermic chemical reaction, resulting in heat release or absorption respectively. Such gel or other medium can include, without limitation: sodium acetate, sodium chloride and water, ammonium nitrate and water, or any other suitable composition. Suitable materials and devices useful for delivering a temperature altering regimen will be obvious to use in conjunction with the current invention, and one of ordinary skill in the art will readily do so without exceeding the scope of the current invention.

If the temperature altering device 18 is removably located in cavity 16, then said temperature altering device 18 is removed from the cavity through sealable pocket mouth 22; is brought to a desired temperature; is returned to cavity 16; sealable pocket mouth 22 is sealed; and a temperature altering regimen is delivered to strategic locations on the horse's body.

Removing and replacing temperature altering device is difficult when sealable pocket mouth 22 is located on the interior side 4 of horse blanket 2 when the horse blanket 2 is on the horse. Cavities 16a-c located at or near the spine and spinal muscles of the horse may be impossible to access when the horse blanket 2 is on the horse.

As sealable pocket mouth 22 is on the interior side 4 of horse blanket 2, it is preferable that the activated temperature altering device is placed within cavity 16 and then horse blanket 2 is placed on the horse's body.

Various modifications and alterations of the invention will become apparent to those skilled in the art without departing from the invention, which is defined by the accompanying claims.

## Claims

1. A temperature altering system, comprising:
a blanket (2) sized and dimensioned to drape over a horse;
first and second pockets (16B,C,D,E) disposed on an underside (4) of the blanket (2), each of which has a cavity that includes a removable temperature altering device (18), **characterised in that** each of the first and second pockets is freely positionable about the blanket using hook and loop fasteners; and **in that**
the first pocket (16B,16C) has a first size and the second pocket (16D,16E) has a second size that is different from the first size; and **in that** a flap (12) is coupled to the blanket (2), wherein the flap (12) includes another pocket (16F) positioned to deliver a temperature altering regimen to a stifle joint of the horse.

2. The system of claim 1, wherein the underside (4) of the blanket (2) includes at least one of a wicking material and a temperature reflective material.

3. The system of claim 1, wherein the first pocket (16B,16C) has a flap disposed to assist in keeping a corresponding one of the temperature altering devices (18) within a cavity of the first pocket.

4. The system of claim 1, wherein the first pocket (16B,16C) has a zipper disposed to assist in keeping a corresponding one of the temperature altering devices (18) within a cavity of the first pocket.

5. The system of claim 1, wherein the first pocket (16B,16C) has a button disposed to assist in keeping a corresponding one of the temperature altering devices (18) within a cavity of the first pocket.

6. The system of claim 1, wherein the pockets (16) mate with a top side (6) of the blanket.

7. The system of claim 1, wherein the blanket further comprises a detachable neck protrusion (10), wherein the protrusion (10) includes an additional pocket (16A,B,C).

8. The system of claim 1, wherein the first size and dimension of the first pocket (16B,16C) is configured to contact the horse's spinal muscles.

9. The system of claim 1, wherein the second size and dimension of the second pocket (16E,16D) is configured to contact at least one of the horse's shoulder and hip muscles.

10. The system of claim 1, wherein the blanket further comprises a row of fixed pockets (16A, 16B, 16C) positioned to deliver the temperature altering regimen to a horse's spinal muscles.

## Patentansprüche

1. Ein Temperaturänderungssystem umfassend:
eine Decke (2) ausgemessen und dimensioniert um ein Pferd zu bedecken;
erste und zweite Taschen (16B,C,D,E) angeordnet an einer Unterseite (4) der Decke (2), wobei jede der beiden einen Hohlraum hat, der eine auswechselbare Temperaturänderungsvorrichtung (18) enthält, **dadurch gekennzeichnet, dass** jede der ersten und zweiten Taschen frei über die Decke positionierbar ist unter Verwendung von Klettverschlüssen; und dass die erste Tasche (16B, 16C) eine erste Grösse hat und die zweite Tasche (16D,16E) eine zweite Grösse hat, die sich von der ersten Grösse unterscheidet; und dass eine Lasche (12) mit der Decke (2) verbunden ist, wobei die Lasche (12) eine weitere Tasche (16F) enthält, die angeordnet ist um eine Temperatur verändernde Kur an ein Kniegelenk des Pferdes abzugeben.

2. Das System gemäss Anspruch 1, wobei die Unterseite (4) der Decke (2) mindestens eines der Materialien umfasst, ein saugfähiges Material und ein Temperatur reflektierendes Material.

3. Das System gemäss Anspruch 1, wobei die erste Tasche (16B, 16C) eine Lasche hat, welche angeordnet ist um zu helfen eine der entsprechenden Temperaturänderungsvorrichtungen (18) innerhalb eines Hohlraumes der ersten Tasche zu halten.

4. Das System gemäss Anspruch 1, wobei die erste Tasche (16B, 16C) einen Reissverschluss hat, welche angeordnet ist um zu helfen eine der entsprechenden Temperaturänderungsvorrichtungen (18) innerhalb eines Hohlraumes der ersten Tasche zu halten.

5. Das System gemäss Anspruch 1, wobei die erste Tasche (16B, 16C) einen Knopf hat, angeordnet um zu helfen eine einzelne der entsprechenden Temperaturänderungsvorrichtungen (18) innerhalb eines Hohlraumes der ersten Tasche zu halten.

6. Das System gemäss Anspruch 1, wobei die Taschen (16) sich an eine Oberseite (6) der Decke anfügen.

7. Das System gemäss Anspruch 1, wobei die Decke zudem ein ablösbares vorstehendes Halsteil (10) umfasst, wobei das vorstehende Teil (10) eine zusätzliche Tasche (16A,B,C) umfasst.

8. Das System gemäss Anspruch 1, wobei die erste Grösse und Dimension der ersten Tasche (16B, 16C) ausgestaltet ist die Rückenmuskeln des Pferdes zu berühren.

9. Das System gemäss Anspruch 1, wobei die zweite Grösse und Dimension der zweiten Tasche (16E, 16D) ausgestaltet ist um zumindest einen der Schulter- und Hüftmuskeln des Pferdes zu berühren.

10. Das System gemäss Anspruch 1, wobei die Decke weiter eine Reihe von festen angemachten Taschen (16A, 16B, 16C) aufweist, die angeordnet sind um die Temperaturänderungskur auf die Rückenmuskeln des Pferdes zu übertragen.

## Revendications

1. Système de modification de température, comprenant :
une couverture (2) taillée et dimensionnée pour recouvrir un cheval,
des première et deuxième poches (16B, C, D, E) disposées sur un côté intérieur (4) de la couverture (2), dont chacune présente une cavité qui comprend un dispositif de modification de température (18) détachable, **caractérisé en ce que** chacune des première et deuxième poches peut être positionnée librement autour de la couverture à l'aide de moyens de fixation par crochets et boucles et **en ce que**
la première poche (16B, 16C) présente une première taille et la deuxième poche (16D, 16E) présente une deuxième taille qui est différente de la première taille, et **en ce que**
un rabat (12) est relié à la couverture (2), où le rabat (12) comprend une autre poche (16F) positionnée pour délivrer un régime de modification de température à une articulation de grasset du cheval.

2. Système selon la revendication 1, dans lequel le côté intérieur (4) de la couverture (2) comprend au moins l'un d'un matériau de dissipation et d'un matériau thermo-réfléchissant.

3. Système selon la revendication 1, dans lequel la première poche (16B, 16C) comporte un rabat disposé pour aider à maintenir un dispositif correspondant parmi les dispositifs de modification de température (18) à l'intérieur d'une cavité de la première poche.

4. Système selon la revendication 1, dans lequel la première poche (16B, 16C) comporte une fermeture à glissière disposée pour aider à maintenir un dispositif correspondant parmi les dispositifs de modification de température (18) à l'intérieur d'une cavité de la première poche.

5. Système selon la revendication 1, dans lequel la première poche (16B,16C) comporte un bouton disposé pour aider à maintenir un dispositif correspondant parmi les dispositifs de modification de température (18) à l'intérieur d'une cavité de la première poche.

6. Système selon la revendication 1, dans lequel les poches (16) s'adaptent à un côté supérieur (6) de la couverture.

7. Système selon la revendication 1, dans lequel la couverture comprend en outre une partie protubérante pour encolure détachable (10), dans lequel la partie protubérante (10) comprend une poche supplémentaire (16A, B, C).

8. Système selon la revendication 1, dans lequel la première taille et la première dimension de la première poche (16B, 16C) sont configurées pour venir au contact des muscles spinaux du cheval.

9. Système selon la revendication 1, dans lequel la deuxième taille et la deuxième dimension de la deuxième poche (16E, 16D) sont configurées pour venir au contact avec au moins l'un des muscles des épaules et des muscles des hanches du cheval.

10. Système selon la revendication 1, dans lequel la couverture comprend en outre une rangée de poches fixées (16A, 16B, 16C) positionnées pour délivrer le régime de modification de température aux muscles spinaux d'un cheval.
